# EUROPÄISCHE PATENTSCHRIFT

(11) **EP 1 103 769 B1**
(45) Veröffentlichungstag und Bekanntmachung des Hinweises auf die Patenterteilung: **11.08.2004**
(21) Anmeldenummer: 00125614.8
(22) Anmeldetag: 23.11.2000
(51) Int. Cl.: F25B 1/00, F25B 41/04

(54) **Analysengas-Kühlvorrichtung**
Refrigerating device for gases of analysis
Dispositif de refroidissement pour gaz d'analyse

(30) Priorität: 27.11.1999 DE 19957052
(43) Veröffentlichungstag der Anmeldung: 30.05.2001
(73) Patentinhaber: M & C Products Analysentechnik GmbH, 40885 Ratingen (DE)
(72) Erfinder: Gawlik, Helmut, 40878 Ratingen (DE)
(74) Vertreter: Becker, Thomas, Dr., Dipl.-Ing.

(56) Entgegenhaltungen:
- EP-A- 0 523 902
- DE-A- 2 656 664
- DE-C- 968 680
- DE-U- 9 111 038
- DE-U- 9 315 705
- US-A- 2 224 377
- US-A- 2 291 503
- US-A- 4 364 238

## Beschreibung

Die Erfindung bezieht sich auf eine Verwendung nach Anspruch 1.

Zu analysierende Gasproben sollen zu ihrer Analyse auf einer konstanten Temperatur gehalten werden. Dieses ist beispielsweise wichtig, um den Taupunkt des Analysengases, also die Temperatur, bei der der Partialdruck des darin enthaltenen Wasserdampfes gleich dessen Sättigungsdampfdruck ist, einstellen zu können. Hierfür ist das Kältemittel der Kühlvorrichtung auf einer möglichst exakt konstant gehaltenen Temperatur zu halten. Diese Temperatur liegt typischerweise im Bereich zwischen 0 und 10°C. Das Regelungsintervall beträgt im Stand der Technik beispielsweise +/- 1,0°C. Da die zu analysierenden Gase nur in Proben von geringen Volumina untersucht werden, benötigt die Kühlvorrichtung nur eine geringe Kühlleistung, beispielsweise im Bereich von 100 Watt oder weniger.

Das Analysengas steht mit der Kühlvorrichtung beispielsweise über Kühlschlangen in Verbindung, die von der Kühlvorrichtung nach Art eines Wärmetauschers auf einer konstanten Temperatur gehalten werden sollen.

Zur Bewirkung der Temperaturkonstanthaltung ist es bekannt, einen im Kühlkreislauf enthaltenen Verdichter temperaturgeregelt ein- oder auszuschalten. Durch das Ausschalten des Verdichters bei Erreichen einer unteren Grenztemperatur wird die Zufuhr von zunächst verflüssigtem Kühlmittel zu einem Verdampfer gestoppt, so daß kein weiterer Wärmeentzug durch die Aggregatzustandsänderung des Kältemittels im Verdampfer mehr stattfinden kann und entsprechend die dort gemessene Temperatur steigt. Nach Erreichen einer oberen Grenztemperatur wird dann der Verdichter wieder eingeschaltet, um eine Herunterregelung der Temperatur zu bewirken und die Temperatur des Analysengases zu vermindern. Dieses bewirkt einen hohen Verschleiß des Verdichters, zudem ist die Genauigkeit einer derartigen Regelung gering, da auch bei ausgeschaltetem Verdichter zunächst noch Kältemittel in den Verdampfer eingespritzt wird.

Der Erfindung liegt das Problem zugrunde, hier eine Verbesserung zu erreichen.

Grundgedanke der Erfindung ist es, die Temperatur an der Kühlvorrichtung kontinuierlich oder getaktet zu messen und in Abhängigkeit von Schwankungen gegenüber einem vorgegebenen Sollwert die Kühlmittelzufuhr zu einem Förderorgan (Verdichter) für das Kühlmittel korrespondierend einzustellen.

Dazu wird erfindungsgemäß die Verwendung eine Kühlvorrichtung mit einem Kreislauf für ein Kältemittel vorgeschlagen, wobei der Kreislauf zumindest einen Verdichter, einen nachgeschalteten Verflüssiger und einen ausgangsseitig mit dem Verdichter verbundenen Verdampfer für das Kältemittel umfaßt, wobei zwischen die Ausgangsseite des Verdampfers und dem Verdichter ein Steuerglied zur Beeinflussung des vom Verdampfer zum Verdichter geleiteten Gasstroms geschaltet ist, das bei Unterschreiten einer unteren Grenztemperatur den Gasstrom zwischen dem Verdampfer (96) und dem Verdichter (2) vermindert oder abschaltet und bei Überschreiten einer oberen Grenztemperatur den Gasstrom zwischen dem Verdampfer (96) und dem Verdichter (2) bis zu einem Maximalwert vergrössert.

Durch die Anordnung eines Steuerglieds zwischen der Ausgangsseite des Verdampfers und dem Verdichter ist eine Einregelung der Temperatur allein über Beeinflussung dieses Steuerglieds möglich. Der Verdichter als förderndes Organ kann hiervon unbeeinflußt durchgehend weiterlaufen, ohne daß er den verschleißfördernden Schaltvorgängen ausgesetzt wäre.

Das Steuerglied kann in einen Regelkreis eingebunden sein und kann von einer am Verdampfer gemessenen Temperatur als Eingangsparameter gesteuert werden. Dadurch ist eine äußere Beeinflussung der Kühlvorrichtung entbehrlich, die Temperatur wird automatisiert konstant gehalten.

Eine Vorrichtung mit den sogenannten Merkmalen ist bekannt aus US 2,291,503 und wird dort als Klimaanlage verwendet.

Bei Verwendung eines Ventils als Steuerglied ergibt sich ein billiges und zuverlässiges Bauteil, das die Unterbrechung beziehungsweise Durchleitung des Gasstroms zwischen dem Verdampfer und dem Verdichter übernimmt.

Entsprechend kann das Ventil den Gasstroms ganz oder teilweise drosseln beziehungsweise freigeben.

Um etwaig vorhandenes Restwasser aus dem Kühlmittel zu entfernen, kann dem Verdampfer eine Trocknungseinheit für das Kältemittel vorgeschaltet sein.

Weitere Vorteile und Merkmale einer Vorrichtung für die erfindungsgemäße Verwendung ergeben sich aus einem in der Zeichnung dargestellten und nachfolgend beschriebenen Ausführungsbeispiel.

In der Zeichnung zeigt:

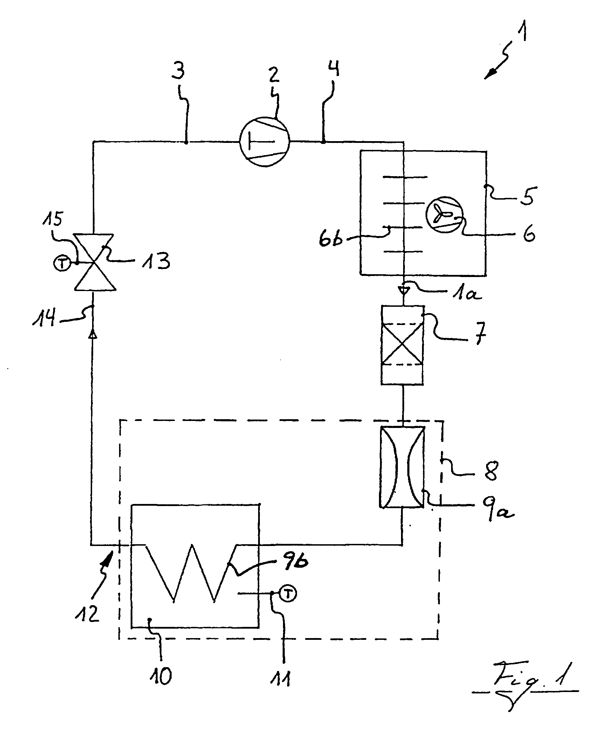

- Figur 1:: ein schematisches Schaltbild einer Kühlvorrichtung zur erfindungsgemäßen Verwendung.

Gemäß dem Ausführungsbeispiel umfaßt die insgesamt mit 1 bezeichnete Analysengas-Kühlvorrichtung in einem Kreislauf 1a einen Verdichter 2, der ein eingangsseitig (bei 3) gefördertes Gas, das einen Druck im Bereich von etwa 0 bis 1 bar aufweist, ausgangsseitig (bei 4) auf einen Druck von etwa 10 bis 15 bar verdichtet.

Das so verdichtete Gas wird einem Verflüssiger 5 zugeleitet, der über ein Kühlgebläse 6 eine Abkühlung des durchgeleiteten Gases vornimmt, wozu insbesondere Kühlrippen 6b zur Vergrößerung der beaufschlagten Oberfläche vorgesehen sein können.

Durch die Abkühlung einerseits und Druckerhöhung andererseits findet eine Verflüssigung des durchgeleiteten Gases statt, das im weiteren einem optional im Kreislauf 1 angeordneten Trockner 7 zugeleitet werden kann, der über Chemi- oder Physisorption eine Wasserabscheidung vornimmt.

Das verflüssigte Kältemittel wird im weiteren einer insgesamt mit 8 bezeichneten Einheit zugeleitet.

Das Kältemittel wird dabei zunächst durch ein Kapillarrohr 9a geführt, wobei der Druck des Kältemittels auf beispielsweise 1-2 bar abgesenkt wird. Es gelangt danach in einen Verdampfer 9b, der in einem Kühlblock 10 liegt, der über den Verdampfer 9b gekühlt wird.

Im Kühlblock 10 ist ein (nicht dargestellter) Wärmetauscher angeordnet, durch den das zu analysierende Gas geführt wird.

Zwischen Verdampfer 9b und dem genannten Wärmetauscher findet entsprechend ein Temperaturausgleich statt.

Das Kältemittel kommt mit dem Wärmetauscher nicht in Kontakt. Es findet auch kein Austausch zwischen dem Analysengas und dem Kältemittelkreislauf 1a statt.

Im Verdampfer 9b wird durch die Änderung des Aggregatzustandes des Kühlmittels von flüssig auf gasförmig die Verdampfungswärme entzogen, die erforderlich ist, um die Aggregatzustandsänderung zu bewirken. Das Analysengas kühlt hierdurch ab.

Im Kühlblock 10 wird über einen Meßfühler 11 die Temperatur T gemessen, wobei die Messung konstant oder getaktet erfolgen kann. Am Ausgang (bei 12) des Verdampfers 9b wird das Kühlmittel einem Steuerglied 13 zugeleitet, das eine Beeinflussung des Gasvolumenstroms zwischen dem Verdampfer 9b und der Eingangsseite 3 des Verdichters 2 vornimmt.

Im Ausführungsbeispiel ist das Steuerglied 13 als Ventil ausgebildet, das entweder eine vollständige Öffnung oder Sperrung des in einer Leitung 14 geführten Kühlmittels ermöglicht. Es kommen grundsätzlich auch Steuerglieder 13 mit kontinuierlich oder abgestuft variablen Zwischen-Öffnungszuständen in Betracht.

Im Ausführungsbeispiel ist die Kühlvorrichtung 1 insgesamt als Regelkreis ausgebildet, wobei die durch den Meßfühler 11 im Kühlblock 10 erfaßte Temperatur ein Eingangssignal 15 für das Steuerglied 13 bildet.

Während des Betriebes wird bei Erreichen einer oberen Grenztemperatur, gemessen am Temperaturfühler 11, das Ventil 13 auf Durchlaß geschaltet. Hierdurch strömt neues Kältemittel in den Verdampfer 9b, wird dort verdampft und entzieht der Umgebung Wärme, so daß die Temperatur im Verdampfer absinkt, wodurch entsprechend auch das Analysengas am Überschreiten einer Grenztemperatur gehindert wird.

Bei weiterem Absinken der Temperatur gibt bei einer unteren Grenztemperatur der Meßfühler 11 ein Signal an den Eingang 15 des Ventils ab, das ein Sperren des Kältemitteldurchgangs durch dieses Ventil 13 bewirkt. Trotz des Weiterbetriebs des Verdichters 2 kann dieser mangels Gasangebots auf der Eingangsseite 3 kein Kältemittel in den Kreislauf einfördern, so daß am Verdampfer 9b kein weiteres Kältemittel verdampfen kann und entsprechend ein Energieentzug aus der Umgebung und dem Analysengas nicht mehr stattfindet. Dessen Temperatur steigt daher wieder, was schließlich bei Erreichen einer oberen Grenztemperatur, erfaßt durch den Meßfühler 11, zur erneuten Öffnung des Ventils 13 führt. Durch die erneute Zufuhr von Kältemittel in den Verdampfer 8 wird dann wieder die Temperaturabsenkung des Analysengases bewirkt.

Eine derartige Kühlvorrichtung 1 kann eine sehr genaue Temperaturregelung im Bereich von +/- 0,1°C bewirken.

Die Kühlvorrichtung arbeitet unabhängig von der Umgebungstemperatur und berücksichtigt lediglich die am Meßfühler 11 erfaßte Temperatur im Kühlblock 10 beziehungsweise dessen Bestandteilen. Diese Regelung ist auch unabhängig von der abgenommenen Leistung.

## Patentansprüche

1. Verwendung einer Kühlvorrichtung (1) mit einem Kreislauf (1a) für ein Kältemittel, wobei der Kreislauf (1a) zumindest einen Verdichter (2), einen nachgeschalteten Verflüssiger (5) und einen ausgangsseitig (12) mit dem Verdichter (2) verbundenen Verdampfer (9b) für das Kältemittel umfaßt, und wobei zwischen der Ausgangsseite (12) des Verdampfers (9b) und dem Verdichter (2) ein Steuerglied (13) zur Beeinflussung des vom Verdampfer (9b) zum Verdichter (2) geleiteten Gasstromes angeordnet ist das bei Unterschreiten einer unteren Grenztemperatur den Gasstrom zwischen dem Verdampfer (9b) und dem Verdichter (2)vermindert oder abschaltet und bei Überschreiten einer oberen Grenztemperatur den Gasstrom zwischen dem Verdampfer (9b) und dem Verdichter (2) bis zu einem Maximalwert vergrößert, Zur Kühlung von Analysengas.

2. Verwendung der Kühlvorrichtung nach Anspruch 1 mit die Maßgabe, daß der Kreislauf (1a) als Regelkreis ausgebildet ist.

3. Verwendung der Kühlvorrichtung nach Anspruch 1 mit der Maßgabe, daß die Temperatur (T) am Verdampfer (9b) meßbar und als Eingangssignal (15) an das Steuerglied (13) übertragbar ist.

4. Verwendung der Kühlvorrichtung nach Anspruch 1 mit der Maßgabe, daß das Schaltglied (13) ein Ventil ist.

5. Verwendung der Kühlvorrichtung nach Anspruch 1 mit der Maßgabe, daß dem Verdampfer (9b) eine Trocknungseinheit (7) für das Kältemittel vorgeordnet ist.

6. Verwendung der Kühlvorrichtung nach Anspruch 1 mit der Maßgabe, daß dem Verdampfer (9b) eine Kapillareinrichtung (9a) im Kreislauf (1a) vorgeschaltet ist.

7. Verwendung dei Kühlvorrichtung nach Anspruche 1 mit der Maßgabe, daß der Verdampfer (9b) in einem oder um einen Kühlblock (10) angeordnet ist, durch den das Analysengas führbar ist.

## Claims

1. Use of a cooling apparatus (1), comprising a circuit (1a) for a coolant, wherein the circuit (1a) includes at least a compressor (2), a liquefier (5) connected downstream thereof and an evaporator (9b) for the coolant connected at its outlet side (12) to the compressor (2), and wherein a control member (13) is arranged between the outlet side (12) of the evaporator (9b) and the compressor (2) for influencing the flow of gas conducted from the evaporator (9b) to the compressor (2), which control member (13) reduces or switches off the flow of gas between the evaporator (9b) and the compressor (2) if the temperature falls below a lower limit temperature, and increases the flow of 20 gas between the evaporator (9b) and the compressor (2) to a maximum value if an upper limit temperature is exceeded, for cooling analysis gas.

2. Use of the cooling apparatus according to claim 1, provided that the circuit (1a) is configured as a control loop.

3. Use of the cooling apparatus according to claim 1, provided that the temperature (T) at the evaporator (9b) is measurable and can be transmitted to the control element (13) as an input signal (15).

4. Use of the cooling apparatus according to claim 1, provided that the switching element (13) is a valve.

5. Use of the cooling apparatus according to claim 1, provided that a drying unit (7) for the coolant is arranged upstream of the evaporator (9b).

6. Use of the cooling apparatus according to claim 1, provided that a capillary device (9a) is connected upstream of the evaporator (9b) in the circuit (1a).

7. Use of the cooling apparatus according to claim 1, provided that the evaporator (9b) is arranged in or around a cooling block (10) through which the analysis gas can be passed.

## Revendications

1. Utilisation d'un dispositif de refroidissement (1) avec un circuit (1a) pour réfrigérant, le circuit (1a) comprenant au moins un compresseur (2), un condenseur (5) en aval et un évaporateur (9b) pour le réfrigérant relié côté sortie (12) au compresseur (2), et un organe de commande (13), destiné à agir sur le flux de gaz allant de l'évaporateur (9b) au compresseur (2), étant placé entre la sortie (12) de l'évaporateur (9b) et le compresseur (2), qui réduit ou arrête le flux de gaz entre l'évaporateur (9b) et le compresseur (2) si la température descend en dessous d'une limite inférieure définie et qui augmente jusqu'à une valeur maximale le flux de gaz entre l'évaporateur (9b) et le compresseur (2) si la température dépasse une limite supérieure afin de refroidir le gaz d'analyse.

2. Utilisation d'un dispositif de refroidissement selon la revendication 1 sous réserve que le circuit (1a) soit conformé en circuit de régulation.

3. Utilisation d'un dispositif de refroidissement selon la revendication 1 sous réserve que la température (T) soit repérable sur l'évaporateur (9b) et que le signal d'entrée (15) soit transmissible à l'organe de commande (13).

4. Utilisation d'un dispositif de refroidissement selon la revendication 1 sous réserve que l'organe de commande (13) soit une vanne.

5. Utilisation d'un dispositif de refroidissement selon la revendication 1 sous réserve qu'une unité de dessication (7) du réfrigérant soit placée avant l'évaporateur (9b).

6. Utilisation d'un dispositif de refroidissement selon la revendication 1 sous réserve qu'un dispositif capillaire (9a) soit installé sur le circuit (1a) avant l'évaporateur (9b).

7. Utilisation d'un dispositif de refroidissement selon la revendication 1 sous réserve que l'évaporateur (9b) soit placé à l'intérieur ou autour d'un bloc réfrigérant (10) à travers lequel le gaz d'analyse puisse circuler.
